(19) Europäisches Patentamt
European Patent Office
Office européen des brevets

(11) Veröffentlichungsnummer: 0 007 537
B1

(12) # EUROPÄISCHE PATENTSCHRIFT

(45) Veröffentlichungstag der Patentschrift: **03.11.82**

(51) Int. Cl.³: **A 61 K 7/13**

(21) Anmeldenummer: **79102443.3**

(22) Anmeldetag: **16.07.79**

(54) **Mittel zur Färbung von Haaren.**

(30) Priorität: **20.07.78 DE 2831847**

(43) Veröffentlichungstag der Anmeldung:
**06.02.80 Patentblatt 80/3**

(45) Bekanntmachung des Hinweises auf die Patenterteilung:
**03.11.82 Patentblatt 82/44**

(84) Benannte Vertragsstaaten:
**AT BE CH DE FR GB IT NL SE**

(56) Entgegenhaltungen:
**DE - A - 2 155 390**
**DE - A - 2 215 303**
**US - A - 2 273 564**
**US - A - 3 820 948**
**US - A - 3 893 803**

Die Akte enthält technische Angaben, die nach dem Eingang der Anmeldung eingereicht wurden und die nicht in dieser Patentschrift enthalten sind.

(73) Patentinhaber: **Wella Aktiengesellschaft**
**Berliner Allee 65**
**D-6100 Darmstadt (DE)**

(72) Erfinder: **Husemeyer, Hans, Dr.**
**Thylmannweg 5**
**D-6100 Darmstadt (DE)**
Erfinder: **Mager, Herbert, Dr.**
**Beaumont 5**
**CH-1700 Fribourg (CH)**
Erfinder: **Konrad, Eugen**
**Mecklenburger Strasse 101**
**D-6100 Darmstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 007 537 B1

# Mittel zur Färbung von Haaren

Die vorliegende Erfindung betrifft Mittel zur Färbung von Haaren auf der Basis von 1-Hydroxymethyl-2,5-diaminobenzol.

Es sind bereits Haarfärbemittel bekannt, welche a) eine bestimmte Guanidinverbindung oder b) Arginin, Protaminproteine oder Polypeptide oder die mit Peroxiden verträglichen Salze dieser Bestandteile in Kombination mit einer wasserlößlichen Peroxidverbindung sowie einem oder mehreren Oxydationsfarbstoff-Vorläufern enthalten. Als Oxydationsfarbstoff-Vorläufer werden unter anderem auch Diaminobenzolderivate der allgemeinen Formel (A) angeführt. Die Haarfärbemittel werden als im wesentlichen geruchsfrei und besonders geeignet hinsichtlich der Beständigkeit der Farbstoffe gegen Shampoos beschrieben (vgl. DE—OS 2 215 303).

Ferner sind schon Haarfärbemittel bekannt geworden, die in einem wäßrigen Medium einen Gehalt an einer Mischung von Oxydationsfarbstoffen aus a) bestimmten Pyrazolonen mit b) bestimmten aromatischen polyfunktionalen Aminen oder bestimmten 4-Aminopyrazolonen oder den wasserlößlichen Salzen dieser 4-Aminopyrazolone aufweisen. Also polyfunktionale Amine werden Aminobenzolderivate einer allgemeinen Formel angegeben. Diese Haarfärbemittel sollen die vorteilhaften Eigenschaften besitzen, mit Reduktionsmitteln wieder entfernbare Haarfärbungen zu erzeugen (vgl. US—PS 3 820 948).

Schließlich ist bereits auch bekannt, daß sich bestimmte m-Toluidinverbindungen als Ausgangsprodukte zur Herstellung von Azo-, Anthrachinon- und Indophenol- Farben eignen. Als geeignete m-Toluidinverbindung wird auch das 1 - Hydroxymethyl - 2,5 - diaminobenzol genannt. Die erwähnten Farben werden unter anderem zur Färbung von Textilien auf Zelluloseesterbasis empfohlen (vgl. US—PS 2 273 564).

Die besonders vorteilhaften Eigenschaften des ausgewählten 1 - Hydroxymethyl - 2,5 - diaminobenzols als Bestandteil von Haarfärbemitteln gemäß der vorliegenden Erfindung sind aus den vorstehend erläuterten Veröffentlichungen nicht zu entnehmen.

Gegenstand der vorliegenden Erfindung sind Mittel zur oxidativen Färbung von Haaren auf der Basis von 1 - Hydroxymethyl - 2,5 - diaminobenzol als Entwicklersubstanz.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

Als Entwicklersubstanzen werden insbesondere 2,5-Diaminotoluol, p-Aminophenol und 1,4-Diaminobenzol eingesetzt. Von den vorzugsweise verwendeten Kupplersubstanzen sind Resorcin, 4-Chlorresorcin, $\alpha$-Naphthol, m-Aminophenol und Derivate des m-Phenylendiamins zu nennen.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und Färbungen in der gewünschten Intensität ermöglichen. Außerdem ist es erforderlich, daß durch Kombination geeigneter Entwickler- und Kupplerkomponenten eine breite Palette verschiedener Farbnuancen erzeugt werden kann. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben.

Die zur Zeit in Haarfärbemitteln verwendeten sowie auch die in neuerer Zeit empfohlen Entwicklersubstanzen, wie beispielsweise Pyrimidinderivate, können jedoch die vorstehend genannten Anforderungen noch nicht völlig zufriedenstellend erfüllen.

Es bestand daher die Aufgabe, eine geeignete Entwicklersubstanz aufzufinden, die den erwähnten Anforderungen weitestgehend gerecht wird.

Hierzu wurde nun gefunden, daß Mittel zur oxidativen Färbung von Haaren, welche dadurch gekennzeichnet sind, daß sie als Entwicklersubstanz 0,01 bis 3,0 Gewichtsprozent 1 - Hydroxymethyl - 2,5 - diaminobenzol der Formel

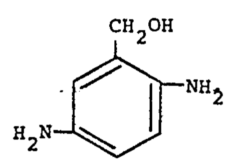

oder dessen Salze mit anorganischen oder organischen Säuren sowie gegebenenfalls übliche Kupplersubstanzen, direktziehende Farbstoffe und/oder Antioxidantien enthalten, in hervorragendem Maße geeignet sind.

Von den üblichen Kupplersubstanzen kommen als Bestandteil der erfindungsgemäßen Haarfärbemittel beispielsweise $\alpha$-Naphthol, 3,4-Diaminobenzoesäure, Resorcin, 4-Chlorresorcin, m-Aminophenol, m-Phenylendiamin, m-Toluylendiamin, 2,4-Diaminoanisol, 2,4-Diaminobenzylalkohol und 3 - Amino - 6 - methylphenol oder Gemische davon in Betracht.

Das als Entwicklersubstanz enthaltene 1 - Hydroxymethyl - 2,5 - diaminobenzol der angegebenen Formel ist gut in Wasser löslich und weist eine ausgezeichnete Lagerbeständigkeit, insbesondere als Bestandteil der erfindungsgemäßen Haarfärbemittel, auf.

In den Haarfärbemitteln soll diese Entwicklersubstanz in einer Konzentration von 0,01 bis 3,0 Gewichtsprozent, insbesondere 0,1 bis 3,0 Gewichtsprozent, enthalten sein. Die Gesamtmenge der Oxidationsfarbstoffe, bestehend aus der Entwicklersubstanz und den üblichen Kupplersubstanzen, beträgt zweckmäßigerweise 0,1 bis 5,0 Gewichtsprozent, insbesondere 0,5 bis 3,0 Gewichtsprozent.

Die Entwicklerkomponente wird im allgemeinen in etwa äquimolaren Mengen, bezogen auf die Kupplerkomponenten, eingesetzt. Es ist jedoch nicht nachteilig, wenn die Entwicklerkomponente diesbezüglich in einem gewissen Überschuß oder Unterschuß vorhaben ist.

Darüber hinaus können die Haarfärbemittel der vorliegenden Anmeldung auch andere bekannte und gebräuchliche Entwicklersubstanzen sowie ferner übliche direktziehende Farbstoffe im Gemisch enthalten, falls dies zur Entwicklung gewisser Farbnuancen notwendig ist.

Außerdem können in den Haarfärbemitteln noch weitere übliche kosmetische Zusätze, beispielsweise Antioxidantien wie Ascorbinsäure oder Natriumsulfit, Parfümöle, Komplexbildner, Netzmittel, Emulgatoren, Verdicker, Pflegestoffe und andere, enthalten sein.

Die Zubereitungsform kann aus einer Lösung, vorzugsweise aus einer Creme, einen Gel oder einer Emulsion, bestehen. Ihre Zusammensetzung stellt eine Mischung der Farbstoffkomponenten mit den für solche Zubereitungen üblichen Bestandteilen dar. Als übliche Bestandteile von Cremes, Emulsionen oder Gelen kommen beispielsweise Netzmittel oder Emulgatoren aus den Klassen der anionischen oder nichtionogenen oberflächenaktiven Substanzen wie Fettalkoholsulfate, Fettsäurealkanolamide, Alkylsulfonate, Alkylbenzolsulfonate, oxäthylierte Fettalkohole, oxäthylierte Nonylphenole, ferner Verdicker wie höhere Fettalkohole, Stärke, Cellulosederivate, Paraffinöl und Fettsäuren sowie außerdem Pflegestoffe wie Lanolinderivate, Cholesterin und Pantothensäure in Betracht.

Die erwähnten Bestandteile werden in den für solche Zwecke üblichen Mengen verwendet, z.B. die Netzmittel und Emulgatoren in Konzentrationen von 0,5 bis 30 Gewichtsprozent, während die Verdicker in einer Menge von 0,1 bis 25 Gewichtsprozent in den Zubereitungen enthalten sein können.

Je nach Zusammensetzung können die erfindungsgemäßen Färbemittel schwach sauer, neutral oder alkalisch reagieren. Insbesondere weisen sie einen pH-Wert im alkalischen Bereich zwischen 8,0 und 11,5 auf, wobei die Einstellung vorzugsweise mit Ammoniak erfolgt. Es können dazu aber auch organische Amine, z.B. Monoäthanolamin oder Triäthanolamin, Verwendung finden.

Ihre Anwendung erfolgt in bekannter Weise, indem man die Haarfärbemittel kurz vor dem Gebrauch mit einem Oxidationsmittel vermischt und das Gemisch auf das Haar aufträgt. Als Oxidationsmittel zur Entwicklung der Haarfärbung kommt hauptsächlich Wasserstoffperoxid, beispielsweise als 6%ige Lösung, bzw. dessen Additionsverbindungen an Harnstoff, Melamin oder Natriumborat in Betracht. Die Anwendungstemperaturen liegen im Bereich von 15 bis 50°C.

Nach einer Einwirkungszeit von 10 bis 45 Minuten, vorzugsweise etwa 30 Minuten, wird das Haar mit Wasser ausgespült und getrocknet. Gegebenenfalls wird im Anschluß an diese Spülung mit einem Shampoo gewaschen und mit einer schwachen organischen Säure, wie z.B. Zitronensäure oder Weinsäure, nachgespült.

Die Herstellung des erfindungsgemäß verwendeten 1 - Hydroxymethyl - 2,5 - diaminobenzols ist bereits aus der Literatur bekannt und kann auf verschiedene Weise durchgeführt werden. So kann man beispielsweise von o-Aminobenzylalkohol ausgehen. Dieser wird zunächst acetyliert und anschließend nitriert. Durch katalytische Hydrierung der entstandenen Nitroverbindung und folgende Entacetylierung ist das genannte 1 - Hydroxymethyl - 2,5 - diaminobenzol zugänglich.

Die erfindungsgemäßen Haarfärbemittel auf der Basis von 1 - Hydroxymethyl - 2,5 - diaminobenzol als Entwicklersubstanz führen zu Haarfärbungen mit ausgezeichneten Echtheitseigenschaften, insbesondere was die Licht-, Wasch- und Reibechtheit anbetrifft, und lassen sich mit Reduktionsmitteln wieder abziehen.

Von besonderer Bedeutung ist weiterhin der durch die Verwendung des 1 - Hydroxymethyl - 2,5 - diaminobenzols in den Haarfärbemitteln gemäß vorliegender Anmeldung in toxikologischer und dermatologischer Hinsicht erzielte Fortschritt, welcher auf der am substituierten Benzolkern gebundenen Hydroxymethylgruppe und der damit verbundenen Verminderung der Lipoidlöslichkeit beruht.

Hinsichtlich der färberischen Möglichkeiten bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus.

Hinsichtlich der färberischen Möglichkeiten bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus.

Dies wird insbesondere deutlich bei Vergleich von Haarfärbemitteln, die als Entwicklersubstanz einerseits das bekannte 2,5-Diaminotoluol und andererseits das anmeldungsgemäße 1 - Hydroxymethyl - 2,5 - diaminobenzol enthalten. Während bei der oxidativen Färbung von Haaren mit der Entwickler-/Kuppler-Kombination 2,5 - Diaminotoluol/3 - Amino - 6 - methylphenol ein Purpurton erhalten wird, entsteht mit der Kombination 1 - Hydroxymethyl - 2,5 - diaminobenzol/3 - Amino - 6 - methylphenol ein wesentlich farbsatterer purpur-violetter Farbton. In analoger Weise beobachtet man eine Farbvertiefung, wenn man statt der Kombination 2,5-Diaminotoluol/m-Aminophenol die Kombination 1 - Hydroxymethyl - 2,5 - diaminobenzol/m - Aminophenol verwendet.

Die überlegenen färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten, Haaren problemlos und mit bisher nicht erreichter Deckkraft ermöglichen.

In den nachstehenden Beispielen soll der Gegenstand der Erfindung näher erläutert werden.

Beispiele

Beispiel 1

Haarfärbemittel in Gelform

| | | |
|---|---|---|
| 0,25 | g | 1-Hydroxymethyl-2,5-diaminobenzol |
| 0,15 | g | Natriumsulfit, wasserfrei |
| 2,50 | g | Laurylalkohol-diglykoläthersulfat, 28%ige Lösung |
| 0,50 | g | Hydroxyäthylcellulose, hochviskos |
| 5,00 | g | Ammoniak, 22%ig |
| 41,60 | g | Wasser |
| 50,00 | g | |

10 g des obigen Haarfärbemittels werden kurz vor dem Gebrauch mit 10 ml Wasserstoffperoxidlösung (6%ig) gemischt und das Gemisch anschließend auf blonde Naturhaare aufgetragen. Nach einer Einwirkungszeit von 30 Minuten bei 40°C wird mit Wasser gespült und getrocknet. Das Haar hat eine modische violettstichige, beige-blonde Färbung erhalten.

Beispiel 2

Haarfärbemittel in Gelform

| | | |
|---|---|---|
| 0,35 | g | 1-Hydroxymethyl-2,5-diaminobenzol |
| 0,27 | g | Resorcin |
| 0,30 | g | Ascorbinsäure |
| 15,00 | g | Ölsäure |
| 7,00 | g | Isopropanol |
| 10,00 | g | Ammoniak, 22%ig |
| 67,08 | g | Wasser |
| 100,00 | g | |

Man vermischt kurz vor dem Gebrauch 10 g dieses Haarfärbemittels mit 10 ml Wasserstoffperoxidlösung (6%ig) und läßt das Gemisch 30 Minuten lang bei 40°C auf blonde Naturhaare einwirken. Sodann wird mit Wasser gespült und getrocknet. Das Haar ist in einem sehr natürlichen dunkelblonden Farbton gefärbt.

Beispiel 3

Haarfärbemittel in Cremeform

| | | |
|---|---|---|
| 0,35 | g | 1-Hydroxymethyl-2,5-diaminobenzol |
| 0,27 | g | m-Phenylendiamin |
| 15,00 | g | Cetylalkohol |
| 0,30 | g | Natriumsulfit, wasserfrei |
| 3,50 | g | Laurylalkohol-digylkoläthersulfat, 28%ige wäßrige Lösung |
| 3,00 | g | Ammoniak, 22%ig |
| 77,58 | g | Wasser |
| 100,00 | g | |

10 g dieses Haarfärbemittels werden kurz vor dem Gebrauch mit 10 ml Wasserstoffperoxidlösung (6%ig) vermischt. Anschließend trägt man das Gemisch auf blonde Naturhaare auf und läßt 30 Minuten lang bei 40°C einwirken. Danach wird mit Wasser gespült und getrocknet. Das Haar hat eine satte Blaufärbung erhalten.

Beispiel 4

Haarfärbelösung

| | | |
|---|---|---|
| 1,00 | g | 1-Hydroxymethyl-2,5-diaminobenzol |
| 0,60 | g | Resorcin |
| 0,30 | g | m-Aminophenol |
| 0,10 | g | m-Phenylendiamin |
| 10,00 | g | Laurylalkohol-diglykoläthersulfat, 28%ige wäßrige Lösung |
| 10,00 | g | Ammoniak, 22%ig |
| 78,00 | g | Wasser |
| 100,00 | g | |

Man vermischt kurz vor dem Gebrauch 10 g des vorstehenden Haarfärbemittels mit 10 ml Wasserstoffperoxidlösung (6%ig) und läßt die Mischung 30 Minuten lang bei 40°C auf blonde Naturhaare einwirken. Sodann wird mit Wasser gespült und getrocknet. Das Haar ist in einem satten schwarz-braunen Farbton gefärbt.

Alle in der vorliegenden Anmeldung angegebenen Prozentzahlen stellen Gewichtsprozente dar.

## Patentansprüche

1. Mittel zur oxidativen Färbung von Haaren, dadurch gekennzeichnet, daß es als Entwicklersubstanz 0,01 bis 3,0 Gewichtsprozent 1 - Hydroxymethyl - 2,5 - diaminobenzol der Formel

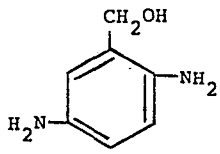

oder dessen Salze mit anorganischen oder organischen Säuren sowie gegebenenfalls übliche Kupplersubstanzen, direktziehende Farbstoffe und/oder Antioxidantien enthält.

2. Mittel nach Anspruch 1, dadurch gekennzeichnet, daß es als Kupplersubstanzen $\alpha$-Naphthol, 3,4-Diaminobenzoesäure, Resorcin, 4-Chlorresorcin, m-Aminophenol, m-Phenylendiamin, m-Toluylendiamin, 2,4-Diaminoanisol, 2,4-Diaminobenzylalkohol und 3 - Amino - 6 - methylphenol oder Gemische davon enthält.

3. Mittel nach Anspruch 1 und 2, dadurch gekennzeichnet, daß es die Entwicklersubstanz in einer Konzentration von 0,1 bis 3,0 Gewichtsprozent enthält.

4. Mittel nach Anspruch 1 bis 3, dadurch gekennzeichnet, daß die Gesamtmenge der Entwickler- Kupplersubstanz-Kombination 0,1 bis 5,0 Gewichtsprozent, vorzugsweise 0,5 bis 3,0 Gewichtsprozent, beträgt.

## Revendications

1. Composition pour la teinture par oxidation des cheveux, caractérisée en ce qu'elle renferme, à titre de substance de développement, de 0.01 à 3.0% en poids de 1 - hydroxyméthyl - 2,5 - diaminobenzène de formule

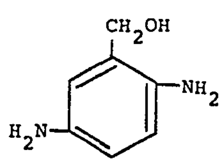

ou ses sels avec des acides inorganiques ou organiques, ainsi que le cas échéant des substances de condensation, des colorants à action direct et/ou des anti-oxydants usuels.

2. Composition suivant la revendication 1, caractérisée en ce qu'elle renferme, comme substances de condensation, de l'$\alpha$-naphtol, de l'acide 3,4-diaminobenzoique, de la résorcine, de la 4-chlororésorcine, du m-aminophénol, de la m-phénylènediamine, de la m-toluylènediamine, du 2,4-diaminoanisol, de l'alcool 2,4-diamino-benzylique et du 3 - amino - 6 - méthylphénol ou des mélanges de ces composés.

3. Composition suivant les revendications 1 ou 2, caractérisée en ce qu'elle renferme la substance de développement selon une concentration allant de 0.1 à 3.0% en poids.

4. Composition suivant l'une quelconque des revendications 1 à 3, caractérisée en ce que la quantité totale de combinaisons de substances de développement et de substances de condensation représente de 0.1 à 5.0% en poids et de préferénce de 0.5 à 3.0% en poids.

## Claims

1. Medium for the oxidative dyeing of hair, characterised in that it contains as developer substance 0.01 to 3.0 weight percent of 1 - hydroxymethyl - 2,5 - diaminobenzene having the formula

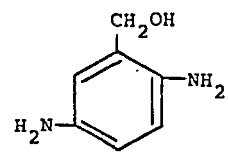

or salts thereof with inorganic or organic acids, and optionally conventional coupler substances, direct dyes, and/or antioxidants.

2. Medium according to Claim 1, characterised in that it contains as coupler substances $\alpha$-naphthol, 3,4-diaminobenzoic acid, resorcinol, 4-chlorresorcinol, m-aminophenol, m-phenylenediamine, m-toluylene diamine, 2,4-diaminoanisol, 2,4-diaminobenzyl alcohol, and 3 - amino - 6 - methylphenol or mixtures thereof.

3. Medium according to Claim 1 or 2, characterised in that it contains the developer substance in a concentration of from 0.1 to 3.0 weight percent.

4. Medium according to any of Claims 1 to 3, characterised in that the total quantity of the developer substance-coupler substance combination amounts to 0.1 to 5.0 weight percent, preferably 0.5 to 3.0 weight percent.